# EUROPÄISCHE PATENTSCHRIFT

(11) **EP 0 262 500 B1**
(45) Veröffentlichungstag und Bekanntmachung des Hinweises auf die Patenterteilung: **18.11.1993**
(21) Anmeldenummer: 87113407.8
(22) Anmeldetag: 14.09.1987
(51) Int. Cl.: A61B 6/14, G05B 19/42

(54) **Zahnärztliche Röntgendiagnostikeinrichtung zur Herstellung von Übersichtsaufnahmen von im Schädel verlaufenden Schichten**
Diagnostic dental X-ray apparatus for producing panoramic exposures of cranial strata
Appareil de radiodiagnostic dentaire pour réaliser des prises panoramiques en couches crâniennes

(30) Priorität: 26.09.1986 DE 3632817
(43) Veröffentlichungstag der Anmeldung: 06.04.1988
(73) Patentinhaber: SIEMENS AKTIENGESELLSCHAFT, 80333 München (DE)
(72) Erfinder: Günther, Werner,, D-6140 Bensheim (DE); Müther, Manfred, D-6140 Bensheim (DE); Heubeck, Erich, D-6140 Bensheim (DE)

(56) Entgegenhaltungen:
- EP-A- 0 035 307
- EP-A- 0 087 946
- DE-A- 3 227 784
- FR-A- 2 151 842
- US-A- 3 283 147
- US-A- 4 039 837

## Beschreibung

Die Erfindung betrifft eine zahnärztliche Röntgendiagnostikeinrichtung entsprechend dem oberbegriff des Patentanspruches 1. ist.

Eine solche Röntgendiagnostikeinrichtung ist beispielsweise aus der DE-A-30 07 935 bzw. EP-A-0 035 307 bekannt. Bei dieser bekannten Einrichtung weist die Eingabeeinrichtung einen mit einer Vielzahl von beim Zubeißen betätigbaren Druckkontakten versehenen, in den Mund eines Patienten einführbaren Körper auf, wobei aus dem Schaltzustand der Druckkontakte Signale für die Steuereinrichtung gewonnen werden, die es gestatten, während der Aufnahme die Bewegungen der Aufnahmeeinheit und des Filmhalters so zu steuern, daß eine durch die betätigten Druckkontakte verlaufende Schicht im Bereich des Kiefers des Patienten aufgenommen wird. Mit der bekannten Röntgendiagnostikeinrichtung können somit unter der Voraussetzung, daß der Patient eine normale Zahnstellung aufweist, Übersichtsaufnahmen guter Qualität von im Bereich des Kiefers des Patienten verlaufenden Schichten angefertigt werden. Bereits wenn der Patient relativ geringfügige Fehlstellungen der Zähne aufweist, ist aber eine einwandfreie Aufnahmequalität im Bereich der Zahnwurzeln nicht mehr mit Sicherheit gewährleistet. Liegen gravierende Fehlstellungen der Zähne vor, besteht aufgrund des Umstandes, daß nur relativ wenige Druckkontakte vorhanden sind, die Gefahr, daß einzelne Zähne des Patienten nicht scharf abgebildet werden. Außerdem können mit der bekannten Röntgendiagnostikeinrichtung Übersichtsaufnahmen von z.B. temporomandibular oder im Bereich der Kieferhöhle verlaufenden Schichten nicht angefertigt werden, da aufgrund der oben beschriebenen Ausbildung der Eingabeeinrichtung der Verlauf der Schicht an die Gebißform des Patienten gebunden ist.

Aus der US-A-3 283 147 ist eine Einrichtung zur Strahlentherapie bekannt, bei der mit Hilfe von z.B. Laserstrahlen chirurgische Eingriffe vorgenommen werden können. Zur Steuerung des Lasers dienen ein oder mehrere Matrixbilder, die vorher vom Objekt, an dem der Eingriff vorgenommen werden soll, aufgenommen werden. Diese Matrixbilder werden zunächst mit Hilfe eines in x-/y-Richtung verstellbaren Lichtzeigers abgetastet. Die entsprechenden Koordinaten werden sodann in einen Computer zur Auswertung eingegeben. Dieser steuert dann den Laser für den Eingriff.

Aus der EP-A-0 087 946 ist ein Verfahren bzw. eine Einrichtung bekannt, die dazu dient, bestimmte Produkte bei deren Fertigung eine geringere Genauigkeit erforderlich ist, leichter herstellen zu können, indem deren Konturen mit Hilfe einer graphischen Eingabeeinrichtung direkt aus einer Zeichnung erfaßt und die hieraus gewonnenen Daten in Form von Koordinaten einer numerisch gesteuerten Werkzeugmaschine zur Herstellung des betreffenden Produktes zugeführt werden. Derartige, aus einer Zeichnung direkt übertragbare Konturen sind beispielsweise Schnittbogen zur Herstellung von Handschuhen, Lederschuhen oder dergleichen.

Der Erfindung liegt eine völlig andere Aufgabe zugrunde, nämlich eine zahnärztliche Röntgendiagnostikeinrichtung der eingangs genannten Art so auszubilden, daß auch bei anomalen Zahnstellungen des Patienten Übersichtsaufnahmen hoher Qualität und solche Übersichtsaufnahmen außerdem unabhängig von der Gebiß- und Kieferform des Patienten von beliebig im Schädel verlaufenden Schichten angefertigt werden können.

Die gestellte Aufgabe wird durch die im Patentanspruch 1 angegebenen Maßnahmen gelöst.

Die erfindungsgemäße Eingabeeinrichtung ist in der Lage, einen als kontinuierlichen Kurvenzug vorliegenden Verlauf der aufzunehmenden Schicht zu erfassen, wobei der Verlauf unabhängig von der Gebiß-und Kieferform und unter Berücksichtigung von Fehlstellungen der Zähne des Patienten beliebig gewählt werden kann.

Die unmittelbar graphische Eingabe kann auf besonders zweckmäßige Weise erfolgen, wenn die Eingabeeinrichtung nach Varianten der Erfindung ein sogenanntes "Computer-Graphics-Tablet" oder einen Monitor mit Lichtgriffel aufweist. In diesen Fällen kann der Verlauf der aufzunehmenden Schicht mittels eines gewöhnlichen Griffels oder Schreibgerätes bzw. des Lichtgriffels unmittelbar auf die berührungsempfindliche Fläche des "Computer-Graphics-Tablet" oder ein auf dieser liegendes Blatt Papier bzw. den Bildschirm des Monitors aufgezeichnet werden, wobei es in beiden Fällen zweckmäßig ist, ein Linienraster vorzusehen, um die Einhaltung gewisser Größenverhältnisse zu gewährleisten. Im Rahmen der Erfindung kann die Eingabeeinrichtung aber auch Mittel, z.B. einen sogenannten Digitizer, aufweisen, die es auf optischem oder mechanischem Wege gestatten, eine Vorlage für den Verlauf der aufzunehmenden Schicht, z.B. einen auf Papier aufgezeichneten, dem Verlauf der aufzunehmenden Schicht entsprechenden Kurvenzug, abzutasten.

Um die optische Kontrolle eines eingegebenen Verlaufes einer aufzunehmenden Schicht zu ermöglichen, weist nach einer Variante der Erfindung die Eingabeeinrichtung Mittel zur graphischen Darstellung von Verläufen aufzunehmender Schichten auf. Falls die Eingabeeinrichtung einen Monitor mit Lichtgriffel aufweist, kann der eingegebene Verlauf auf dem Monitor selbst sichtbar gemacht werden. Weist die Eingabeeinrichtung ein "Computer-Graphics-Tablet" oder andere Mittel zur Eingabe des Verlaufes der aufzunehmenden Schicht auf, können die Mittel zu dessen graphischer Darstellung durch einen zusätzlich vorgesehenen Monitor, Plotter oder Drucker gebildet sein.

Nach einer Ausführungsform der Erfindung ist der Speicher so ausgebildet, daß in ihm Daten mehrerer Verläufe aufzunehmender Schichten speicherbar sind, wobei außerdem ein Teil des Speichers als Nur-Lese-Speicher ausgeführt sein kann, in dem die Daten einer Anzahl von Standardverläufen aufzunehmender Schichten gespeichert sind. Es ist dann möglich, zu bestimmten Patienten gehörige Verläufe aufzunehmender Schichten bei Bedarf wieder abzurufen, um z.B. den Erfolg von kiefer- oder zahnorthopädischen Behandlungen kontrollieren zu können, bzw. im Falle von Patienten, die hinsichtlich ihrer Gebißform und Zahnstellung keine Anomalitäten aufweisen, Übersichtsaufnahmen anzufertigen, ohne daß im Falle eines jeden Patienten ein einem Standardverlauf entsprechender Verlauf einer aufzunehmenden Schicht erneut eingegeben werden müßte. Durch die Speicherung der Standardverläufe in einem Nur-Lese-Speicher wird erreicht, daß diese durch Überschreiben nicht versehentlich verloren gehen können.

Eine besonders komfortable Bedienung der Röntgendiagnostikanlage wird erreicht, wenn die Mittel der Eingabeeinrichtung zur graphischen Darstellung von Verläufen aufzunehmender Schichten derart ausgebildet sind, daß mehrere Verläufe aufzunehmender Schichten gleichzeitig graphisch darstellbar sind und die Eingabeeinrichtung selbst derart ausgebildet ist, daß einer der dargestellten Verläufe aufzunehmender Schichten zur Anfertigung einer entsprechenden Aufnahme unmittelbar wählbar ist. Falls die Eingabeeinrichtung einen Monitor mit Lichtgriffel aufweist, bedeutet dies, daß auf dem Monitor mehrere Verläufe aufzunehmender Schichten dargestellt sind und der für die Anfertigung der folgenden Aufnahme geeignete Verlauf durch Antippen mittels des Lichtgriffels gewählt werden kann.

Um ausgehend von einem eingegebenen Verlauf einer aufzunehmenden Schicht eine entsprechende Übersichtsaufnahme anfertigen zu können, ist nach einer Ausführung der Erfindung eine Recheneinrichtung vorgesehen, welche einen in die Eingabeeinrichtung eingegebenen Verlauf einer aufzunehmenden Schicht in vorzugsweise digitale Daten für die Steuereinrichtung umsetzt. Die Recheneinrichtung kann außerdem dazu dienen, ausgehend von einem Verlauf einer aufzunehmenden Schicht den Verlauf der zugehörigen scharf abbildbaren Schichtdicke zu ermitteln, wobei die Mittel der Eingabeeinrichtung zur graphischen Darstellung von Verläufen aufzunehmender Schichten diesen dann zusätzlich zum eigentlichen Verlauf der aufzunehmenden Schicht darstellen.

Außerdem kann die Recheneinrichtung dazu dienen, ausgehend von einem Verlauf einer aufzunehmenden Schicht diesen im Sinne einer Stauchung und/oder Dehnung zu verarbeiten, wobei die Mittel der Eingabeeinrichtung zur graphischen Darstellung von Verläufen aufzunehmender Schichten den so gewonnenen Verlauf darstellen. Es ist so möglich, bereits eingegebene Verläufe aufzunehmender Schichten zu variieren und den jeweiligen Erfordernissen anzupassen.

In den beigefügten Zeichnungen sind Ausführungsbeispiele der Erfindung dargestellt. Es zeigen:
- Fig. 1: in schematischer perspektivischer Darstellung den mechanischen Aufbau einer Röntgendiagnostikeinrichtung,
- Fig. 2: ein Blockschaltbild einer erfindungsgemäßen Röntgendiagnostikeinrichtung, und
- Fig. 3: ebenfalls ein Blockschaltbild einer erfindungsgemäßen Röntgendiagnostikeinrichtung.

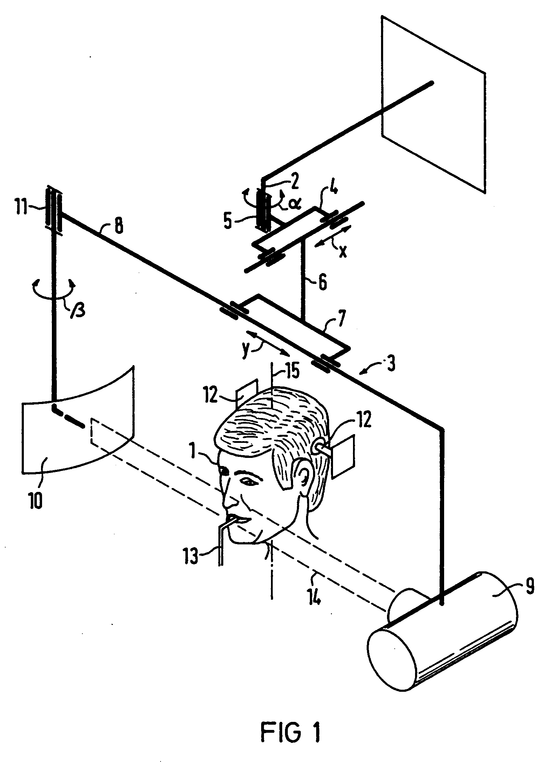

In der Figur 1 ist eine zahnärztliche Röntgendiagnostikeinrichtung zur Herstellung von Übersichtsaufnahmen vom im Schädel 1 eines Patienten, insbesondere im Bereich des Kiefers verlaufenden Schichten dargestellt, welche eine um eine Achse 2 in Richtung des Doppelpfeiles α schwenkbare Aufnahmeeinheit 3 aufweist, die außerdem in einer senkrecht zu der Achse 2 verlaufenden Ebene in Richtung der Doppelpfeile x und y verschiebbar ist. Zu diesem Zweck ist an der Achse 2 eine in einer zu dieser rechtwinklig verlaufenden Ebene angeordnete Längsführung 4 mittels eines Lagers 5 in Richtung des Doppelpfeiles α schwenkbar angebracht. In der Längsführung 4 ist ein Zwischenteil 6 in Richtung des Doppelpfeiles x verschiebbar aufgenommen, das seinerseits eine Längsführung 7 trägt, die um 90° zu der Längsführung 4 versetzt in einer rechtwinklig zu der Achse 2 verlaufenden Ebene angeordnet ist. In der Längsführung 7 ist ein Träger 8 der Aufnahmeeinheit 3 in Richtung des Doppelpfeiles y verschiebbar aufgenommen. Die Längsführungen 4 und 7 sind derart ausgebildet, daß sie ausschließlich Längs- nicht aber Schwenk- oder Drehbewegungen des Zwischenteiles 6 und des Trägers 8 zulassen. An dem Träger 8 ist einerseits eine Röntgenröhre 9 starr angebracht und andererseits ein Filmhalter 10 in einem Lager 11 in Richtung des Doppelpfeiles β schwenkbar aufgenommen. Zwischen der Röntgenröhre 9 und dem dieser gegenüberliegenden etwa halbkreisförmigen Filmhalter 10 ist eine Kopfhalterung für den Schädel 1 des Patienten angeordnet, die Kopfstützen 12 und ein Aufbißteil 13 aufweist und den Schädel des Patienten während der Anfertigung einer Aufnahme stationär haltert.

Zur Anfertigung einer Übersichtsaufnahme wird ein durch eine in Figur 1 nicht sichtbare, vor der Röntgenröhre 9 angeordnete Spaltblende, deren Spalt parallel zu der Achse 2 verläuft, tretendes Röntgenstrahlenbündel durch den Schädel 1 des Patienten geschickt. Zur Erzielung des gewünschten Verlaufes der aufzunehmenden Schicht im Schädel 1 wird dabei die Aufnahmeeinheit 3 in der rechtwinklig zu der Achse 2 verlaufenden Ebene mittels der Längsführungen 4 und 7 in Richtung der Doppelpfeile x und y derart verschoben, daß die in Figur 1 strichliert eingetragene Momentanachse 15, um die die Aufnahmeeinheit gleichzeitig infolge einer Schwenkbewegung der Längsführung 4 um die Achse 2 in Richtung des Doppelpfeiles α geschwenkt wird, eine Bahn beschreibt, die dem Verlauf der aufzunehmenden Schicht entspricht. Die Bewegung der Aufnahmeeinheit 3 erfolgt derart, daß das Röntgenstrahlenbündel 14 den jeweils aufzunehmenden Bereich des Verlaufes der aufzunehmenden Schicht senkrecht durchdringt. Gleichzeitig wird der Filmhalter 10 in entgegengesetzter Richtung zur Schwenkbewegung der Aufnahmeeinheit 3 mit einer Bahngeschwindigkeit geschwenkt, die der jeweiligen Geschwindigkeit der Momentanachse 15 proportional ist, so daß sich eine konstante Vergrößerung längs des Verlaufes der aufzunehmenden Schicht ergibt. Die Bewegungen der Aufnahmeeinheit 3 in den Richtungen der Doppelpfeile x, y und α sowie die Bewegung des Filmhalters 10 in Richtung des Doppelpfeiles β werden durch Schrittmotore, die in Figur 1 der Übersichtlichkeit halber nicht dargestellt sind, bewirkt.

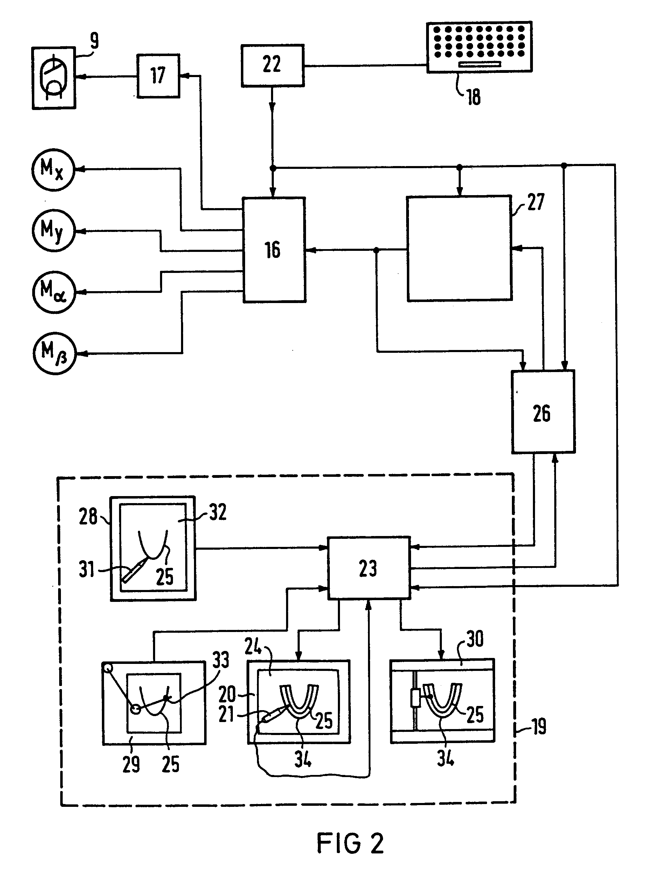

Die Schrittmotore Mₓ, M_{y}, M_{α} und M_{β} sind in Figur 2, die ein Blockschaltbild einer erfindungsgemäßen Röntgendiagnostikanlage zeigt, schematisch dargestellt und werden von einer Steuereinrichtung 16 angesteuert. An diese ist außerdem eine Versorgungseinheit 17 für die Röntgenröhre 9 angeschlossen. Die Steuereinrichtung 16 steht mit einer über eine Tastatur 18 bedienbaren zentralen Steuereinheit 22 in Verbindung. Außerdem weist die erfindungsgemäße Röntgendiagnostikeinrichtung eine Eingabeeinrichtung 19 auf, die u.a. einen Monitor 20 mit Lichtgriffel 21 besitzt, die beide mit einer Eingabe-Ausgabe-Steuerung 23 verbunden sind. Mittels des Lichtgriffels 21 wird auf den Bildschirm 24 des Monitors 20 der Verlauf 25 einer aufzunehmenden Schicht aufgezeichnet, wobei die Eingabe-Ausgabe-Steuerung 23 den Verlauf 25 auf dem Bildschirm 24 des Monitors 20 darstellt. Außerdem übermittelt die Eingabe-Ausgabe-Steuerung 23 dem Verlauf 25 der aufzunehmenden Schicht entsprechende elektrische Signale an eine Recheneinheit 26, die diese in für die Steuereinrichtung 16 geeignete, insbesondere digitale Signale umsetzt und an einen Speicher 27 weitergibt, der diese zunächst speichert. Der Speicher 27 ist mit der Steuereinrichtung 16 und der zentralen Steuereinheit 22 verbunden.

Zur Anfertigung einer Übersichtsaufnahme entsprechend einem eingegebenen Verlauf 25 einer aufzunehmenden Schicht wird derart verfahren, daß zunächst der Schädel 1 des Patienten mittels der Kopfstützen 12 und des Aufbißteiles 13 (Figur 1) in einer für die Aufnahme der gewünschten Schicht geeigneten Lage fixiert wird. Nach geeigneter Betätigung der Tastatur 18 ruft dann die zentrale Steuereinheit 22 die dem Anfangspunkt des Verlaufes 25 der aufzunehmenden Schicht entsprechenden Daten aus dem Speicher 27 ab, die der Steuereinrichtung 16 zugeführt werden, die ihrerseits die Schrittmotore Mₓ, M_{y}, M_{α} und M_{β} derart betätigt, daß die Aufnahmeeinheit 3 und der Filmhalter 10 (Figur 1) eine dem Verlauf 25 der aufzunehmenden Schicht entsprechende Anfangsposition einnehmen. Auf eine weitere geeignete Betätigung der Tastatur 18 hin veranlaßt die zentrale Steuereinheit 22 die Steuereinrichtung 16 dazu, die Röntgenröhre 9 in Betrieb zu setzen. Gleichzeitig bewirkt die zentrale Steuereinrichtung 22 die kontinuierliche Übertragung der dem Verlauf 25 der aufzunehmenden Schicht entsprechenden Daten in die Steuereinrichtung 16, die ihrerseits die Schrittmotore Mₓ, M_{y}, M_{α} und M_{β} derart betätigt, daß die Aufnahmeeinheit 3 und der Filmhalter 10 die zur Anfertigung der Übersichtsaufnahme erforderlichen Bewegungen ausführen. Sobald die Aufnahmeeinheit 3 und der Filmhalter 10 ihre dem Verlauf der aufzunehmenden Schicht entsprechenden Endpositionen erreicht haben, wird die Röntgenröhre 9 außer Betrieb gesetzt.

Die Eingabeeinrichtung 19 der in Figur 2 dargestellten erfindungsgemäßen Röntgendiagnostikanlage weist außer dem Monitor 20 mit Lichtgriffel 21 ein "Computer-Graphics Tablet" 28 und einen Digitizer 29 auf, die wahlweise anstelle des Monitors 20 mit Lichtgriffel 21 zur Eingabe des Verlaufes 25 der aufzunehmenden Schicht zur Verfügung stehen. Auf eine geeignete Betätigung der Tastatur 18 hin veranlaßt die zentrale Steuereinheit 22 die Eingabe-Ausgabe-Steuerung 23 entweder mit dem Monitor 20 mit Lichtgriffel 21, mit dem "Computer-Graphics-Tablet" 28 oder dem Digitizer 29 zur Eingabe des Verlaufes 25 der aufzunehmenden Schicht in Verbindung zu treten. Die Eingabeeinrichtung 19 weist außerdem einen Plotter 30 auf, der auf eine entsprechende Betätigung der Tastatur 18 hin anstelle des Monitors 20 zur graphischen Darstellung des eingegebenen Verlaufes 25 der aufzunehmenden Schicht zur Verfügung steht.

Zur Eingabe eines Verlaufes 25 einer aufzunehmenden Schicht mittels des "Computer-Graphics-Tablet" wird dieser mittels eines Schreibstiftes 31 auf ein auf das "Computer-Graphics-Tablet" 28 aufgelegtes Blatt Papier 32 aufgezeichnet, wobei durch den Druck des Schreibstiftes 31 in der Oberfläche des "Computer-Graphics Tablet" 28 befindliche Drucksensoren betätigt werden, die dem Verlauf 25 der aufzunehmenden Schicht entsprechende elektrische Signale an die Eingabe-Ausgabe-Steuerung 23 geben. Im Falle des Digitizers 29 erfolgt die Eingabe des Verlaufes 25 der aufzunehmenden Schicht dagegen, indem eine graphische Vorlage desselben mittels eines Taststiftes 33 nachgefahren wird, wobei dessen Bewegung in entsprechende elektrische Signale umgesetzt werden, die der Eingabe-Ausgabe-Steuerung 23 zugeführt werden. Von dieser gelangen die dem eingegebenen Verlauf 25 der aufzunehmenden Schicht entsprechenden Signale an die Recheneinrichtung 26, die sie in Daten für die Steuereinrichtung 16 umsetzt und an den Speicher 27 weiterleitet.

Die Recheneinrichtung 26 ist außerdem derart ausgebildet, daß sie in der Lage ist, die zu einem eingegebenen Verlauf 25 einer aufzunehmenden Schicht gehörige scharf abbildbare Schichtdicke zu ermitteln. Zu diesem Zweck ist die Recheneinrichtung 26 zum einen mit dem Ausgang des Speichers 27 und zum anderen mit der zentralen Steuereinheit 22 verbunden, die auf eine geeignete Betätigung der Tastatur 18 hin die in dem Speicher 27 befindlichen Daten des Verlaufes 25 einer aufzunehmenden Schicht der Recheneinrichtung 26 zuführt und diese veranlaßt, den entsprechenden Verlauf 34 der scharf abbildbaren Schichtdicke zu ermitteln und der Eingabe-Ausgabe-Steuerung 23 zuzuführen, die diesen dann auf dem Monitor 20 und/oder dem Plotter 30 darstellt.

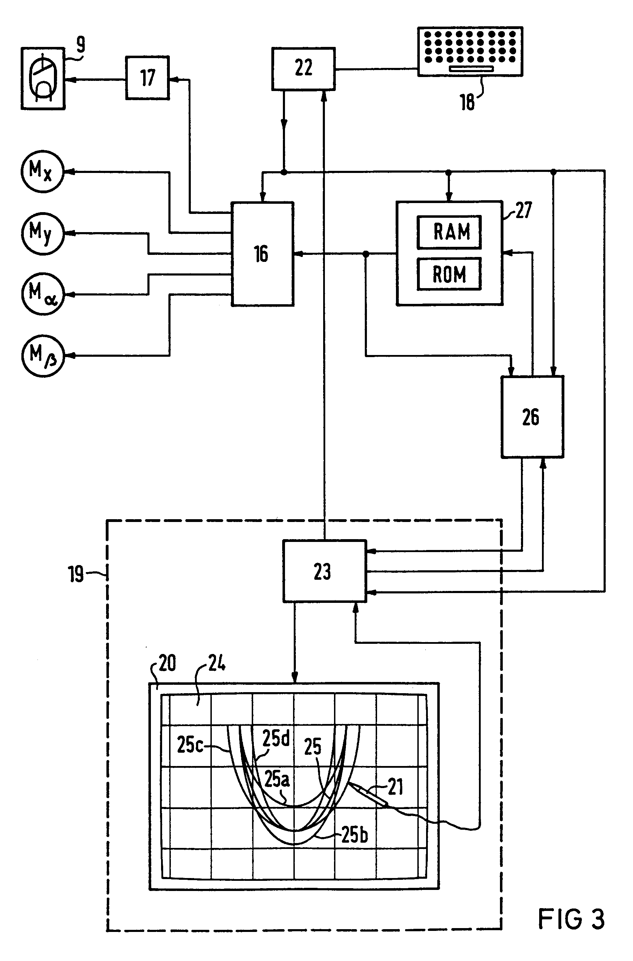

In der Figur 3 ist das Blockschaltbild einer anderen erfindungsgemäßen Röntgendiagnostikeinrichtung dargestellt, die sich von der zuvor beschriebenen zunächst dadurch unterscheidet, daß die Eingabeeinrichtung 19 zur Eingabe und zur graphischen Darstellung eines Verlaufes 25 einer aufzunehmenden Schicht lediglich einen Monitor 20 mit Lichtgriffel 21 aufweist. Außerdem ist der Speicher 27 in zwei Bereiche unterteilt, von denen der eine als Schreib-Lese-Speicher (RAM) und der andere als Nur-Lese-Speicher (ROM) ausgebildet ist, wobei im ersteren die Daten einer Anzahl mittels des Lichtgriffels 21 eingegebener Verläufe 25 aufzunehmender Schichten speicherbar sind, während im letzteren die Daten einer Anzahl von Standardverläufen aufzunehmender Schichten fest gespeichert sind. Desweiteren ist die Recheneinrichtung 26 derart ausgebildet, daß sie die Daten von im Speicher 27 vorhandenen Verläufen 25 aufzunehmender Schichten im Sinne einer Stauchung bzw. einer Dehnung bearbeiten kann. Dies erfolgt auf geeignetes Betätigen der Tastatur 18 hin, worauf die zentrale Steuereinheit 22 die Daten eines bestimmten Verlaufes 25 aus dem Speicher 27 in die Recheneinrichtung 26 überträgt, die diese auf eine mittels der Tastatur 18 wählbare Weise bearbeitet und an die Eingabe-Ausgabe-Steuerung 23 übergibt, die den ursprünglichen Verlauf 25 der aufzunehmenden Schicht gemeinsam mit dem aus diesem abgeleiteten Verlauf, z.B. 25a, auf dem Monitor 20 darstellt. Die Eingabe-Ausgabe-Steuerung 23 kann übrigens durch geeignetes Betätigen der Tastatur 18 veranlaßt werden, mehrere im Speicher 27 vorhandene oder durch die Recheneinrichtung 26 aus solchen abgeleitete Verläufe gleichzeitig darzustellen, was im Falle der Figur 3 beispielhaft an den Verläufen 25, 25a, 25b, 25c und 25d verdeutlicht ist.

Durch Antippen mit dem Lichtgriffel 21 kann einer der auf dem Bildschirm 24 des Monitors 20 dargestellten Verläufe 25a, 25b, 25c, 25d in den Speicher 27 übertragen werden, wo er dann zur Anfertigung einer entsprechenden Übersichtsaufnahme zur Verfügung steht. Die Anfertigung der entsprechenden Übersichtsaufnahme vollzieht sich in der zuvor beschriebenen Weise.

In den Figuren 2 und 3 sind die Steuereinrichtung 16, die Tastatur 18, die Eingabeeinrichtung 19, die zentrale Steuereinheit 22, die Eingabe-Ausgabe-Steuerung 23, die Recheneinrichtung 26 und der Speicher 27 als einzelne Funktionselemente dargestellt. Diese können jedoch auch Bestandteile einer Datenverarbeitungsanlage, z.B. eines Kleincomputers, sein.

## Patentansprüche

1. Zahnärztliche Röntgendiagnostikeinrichtung zur Herstellung von Übersichtsaufnahmen von im Schädel (1) eines Patienten verlaufenden Schichten, insbesondere von im Bereich des Kiefers verlaufenden Schichten, enthaltend
a) eine um eine lotrechte Achse (2 bzw. 15) drehbar angeordnete Aufnahmeeinheit (3), an der eine Röntgenstrahlenquelle (9) und diametral dazu ein relativ zur Strahlenquelle (9) beweglicher Filmhalter (10) gehaltert sind,
b) eine zwischen Strahlenquelle (9) und Filmhalter (10) angeordnete Kopfhalterung (12, 13), mit welcher der Patientenkopf (1) während einer Aufnahme stationär gehaltert werden kann,
c) ein erstes Stellmittel (M_{α} ) zur Verstellung der Aufnahmeeinheit (3) um die lotrechte Achse (2 bzw. 15),
d) ein zweites Stellmittel (M_{β} ) zur Verstellung des Filmhalters (10) relativ zur Strahlenquelle (9),
e) mindestens ein weiteres Stellmittel (Mₓ, M_{y}) zur Verschiebung der Aufnahmeeinheit (3) in einer senkrecht zur lotrechten Achse (2 bzs. 15) verlaufenden Ebene,
f) eine Steuereinrichtung (16) zur Ansteuerung der Strahlenquelle (9) und der vorgenannten Stellmittel (Mₓ, M_{y} sowie M_{α}) und
g) eine Eingabeeinrichtung (19, 23), welche dem Verlauf einer aufzunehmenden Schicht entsprechende elektrische Signale an die Steuereinrichtung (16) liefert,
**dadurch gekennzeichnet,** daß
h) die Eingabeeinrichtung (19, 23) Mittel (20, 21; 28, 31, 32; 29, 33; 30) zur graphischen Eingabe mindestens eines frei wählbaren kontinuierlichen Kurvenzuges einer aufzunehmenden Schicht sowie zur Umsetzung des ausgewählten Kurvenzuges in elektrische Signale enthält, daß
i) ein mit der Eingabeeinrichtung (19, 23) und der Steuereinrichtung (16) verbundener Speicher (27) vorhanden ist, in welchem die aus der graphischen Eingabe erhaltenen elektrischen Signale als Daten abgespeichert werden und daß
j) eine mit der Steuereinrichtung (16) und dem Speicher (27) verbundene und an eine Tastatur (18) angeschlossene zentrale Steuereinheit (22) vorgesehen ist, welche bei Aktivierung durch Abruf der Daten aus dem Speicher (27) die Steuereinrichtung (16) zunächst veranlaßt, die Stellmittel so zu verstellen, daß Aufnahmeeinheit (3) und Filmhalter (10) eine der aufzunehmenden Schicht entsprechende Anfangsposition einnehmen, welche anschließend die Strahlenquelle (9) in Betrieb setzt und dabei entsprechend dem Verlauf der graphischen Eingabe der aufzunehmenden Schicht die Stellmittel so verstellt, daß die Aufnahmeeinheit (3) und der Filmhalter (10) die zur Anfertigung der Übersichtsaufnahme erforderlichen Bewegungen ausführt.

2. Zahnärztliche Röntgendiagnostikeinrichtung nach Anspruch 1**, dadurch gekennzeichnet,** daß die Eingabeeinrichtung (19) ein "Computer-Graphics-Tablet" (28) aufweist.

3. Zahnärztliche Röntgendiagnostikeinrichtung nach Anspruch 1, **dadurch gekennzeichnet,** daß die Eingabeeinrichtung (19) einen Monitor (20) mit Lichtgriffel (21) aufweist.

4. Zahnärztliche Röntgendiagnostikeinrichtung nach einem der Ansprüche 1 bis 3, **dadurch gekennzeichnet,** daß die Eingabeeinrichtung (19) Mittel (20, 30) zur graphischen Darstellung von Verläufen (25, 25a, 25b, 25c, 25d) aufzunehmender Schichten aufweist.

5. Zahnärztliche Röntgendiagnostikeinrichtung nach Anspruch 4, **dadurch gekennzeichnet,** daß der Speicher (27) so ausgebildet ist, daß in ihm Daten mehrerer Verläufe (25) aufzunehmender Schichten speicherbar sind, und daß ein Teil des Speichers (27) als Nur-Lese-Speicher (ROM) ausgeführt ist, in dem die Daten einer Anzahl von Standardverläufen aufzunehmender Schichten gespeichert sind.

6. Zahnärztliche Röntgendiagnostikeinrichtung nach einem der Ansprüche 4 oder 5, **dadurch gekennzeichnet,** daß die Mittel (20) der Eingabeeinrichtung (19) zur graphischen Darstellung von Verläufen (25, 25a, 25b, 25c, 25d) aufzunehmender Schichten derart ausgebildet sind, daß mehrere Verläufe (25, 25a, 25b, 25c, 25d) gleichzeitig graphisch darstellbar sind und die Eingabeeinrichtung (19) selbst derart ausgebildet ist, daß einer der dargestellten Verläufe (25, 25a, 25b, 25c, 25d) aufzunehmender Schichten zur Anfertigung einer entsprechenden Aufnahme unmittelbar wählbar ist.

7. Zahnärztliche Röntgendiagnostikeinrichtung nach einem der Ansprüche 1 bis 6, **dadurch gekennzeichnet,** daß eine Recheneinrichtung (26) vorgesehen ist, welche einen in die Eingabeeinrichtung (19) eingegebenen Verlauf (25) einer aufzunehmenden Schicht in vorzugsweise digitale Daten für die Steuereinrichtung (16) umsetzt.

8. Zahnärztliche Röntgendiagnostikeinrichtung nach den Ansprüchen 4 und 7, **dadurch gekennzeichnet,** daß die Recheneinrichtung (26) ausgehend von einem Verlauf (25) einer aufzunehmenden Schicht, den Verlauf der zugehörigen scharf abbildbaren Schichtdicke (34) ermittelt und die Mittel (20, 30) der Eingabeeinrichtung (19) zur graphischen Darstellung von Verläufen (25, 25a, 25b, 25c, 25d) aufzunehmender Schichten diesen zusätzlich zum eigentlichen Verlauf der aufzunehmenden Schicht (25) darstellen.

9. Zahnärztliche Röntgendiagnostikeinrichtung nach Anspruch 7 oder 8, **dadurch gekennzeichnet,** daß die Recheneinrichtung (26) ausgehend von einem Verlauf (25) einer aufzunehmenden Schicht diesen im Sinne einer Stauchung und/oder Dehnung bearbeitet und die Mittel (20, 30) der Eingabeeinrichtung (19) zur graphischen Darstellung von Verläufen (25, 25a, 25b, 25c, 25d) den so gewonnenen Verlauf (25a, 25b, 25c, 25d) darstellen.

## Claims

1. Dental X-ray diagnostic device for the manufacture of observation photographs of layers extending in the skull (1) of a patient, in particular of layers extending in the region of the jaw, containing
a) a photograph unit (3) rotatably arranged about a vertical axis (2 and 15), on which unit there is supported an X-ray source (9) and there is supported diametrically thereto a film holder (10) movable relative to the radiation source (9),
b) a head support (12, 13) arranged between radiation source (9) and film holder (10), by which the patient's head can be supported in a stationary manner during a photograph,
c) a first adjusting means (M_{α}) for the adjustment of the photograph unit (3) about the vertical axis (2 and 15),
d) a second adjusting means (M_{β}) for the adjustment of the film holder (10) relative to the radiation source (9),
e) at least one further adjusting means (Mₓ, M_{y}) for the displacement of the photograph unit (3) in a plane extending perpendicular to the vertical axis (2 and 15),
f) a control device (16) for the control of the radiation source (9) and the aforementioned adjusting means (Mₓ, M_{y} and M_{α}) and
g) an input device (19, 23), which supplies from the form of a layer to be photographed corresponding electrical signals to the control device (16),
characterized in that
h) the input device (19, 23) contains means (20, 21; 28, 31, 32; 29, 33; 30) for the graphic input of at least one freely selectable continuous curve path of a layer to be photographed and for the conversion of the selected curve path into electrical signals, in that
i) a memory (27) connected to the input device (19, 23) and the control device (16) is present, in which the electrical signals obtained from the graphic input are stored as data and in that
j) a central control unit (22) connected to the control device (16) and the memory (27) and attached to a keyboard (18) is provided, which upon activation, through recall of the data from the memory (27), initially causes the control device (16) to adjust the adjusting means in such a way that the photograph unit (3) and film holder (10) occupy a starting position corresponding to the layer to be photographed, which subsequently puts the radiation source (9) into operation and in this respect corresponding to the form of the graphic input of the layer to be photographed adjusts the adjusting means in such a way that the photograph unit (3) and the film holder (10) carry out the movements necessary for the preparation of the observation photograph.

2. Dental X-ray diagnostic device according to claim 1, characterized in that the input device (19) has a "computer-graphics-tablet" (28).

3. Dental X-ray diagnostic device according to claim 1, characterized in that the input device (19) has a monitor (20) with light pen (21).

4. Dental X-ray diagnostic device according to one of claims 1 to 3, characterized in that the input device (19) has means (20, 30) for the graphic representation of forms (25, 25a, 25b, 25c, 25d) of layers to be photographed.

5. Dental X-ray diagnostic device according to claim 4, characterized in that the memory (27) is constructed in such a way that there can be stored therein data of several forms (25) of layers to be photographed and in that a part of the memory (27) is designed as read-only memory (ROM), in which the data of a number of standard forms of layers to be photographed are stored.

6. Dental X-ray diagnostic device according to one of claims 4 or 5, characterized in that the means (20) of the input device (19) for the graphic representation of forms (25, 25a, 25b, 25c, 25d) of layers to be photographed are constructed in such a way that several forms (25, 25a, 25b, 25c, 25d) can be graphically represented at the same time and the input device (19) itself is constructed in such a way that one of the represented forms (25, 25a, 25b, 25c, 25d) of layers to be photographed can be directly selected for the preparation of an appropriate photograph.

7. Dental X-ray diagnostic device according to one of claims 1 to 6, characterized in that a computing device (26) is provided, which converts a form (25) of a layer to be photographed, entered into the input device (19), into preferably digital data for the control device (16).

8. Dental X-ray diagnostic device according to claims 4 and 7, characterized in that the computing device (26), proceeding from a form (25) of a layer to be photographed, determines the form of the associated sharply imageable layer thickness (34) and the means (20, 30) of the input device (19) for the graphic representation of forms (25, 25a, 25b, 25c, 25d) of layers to be photographed additionally represent these for the actual form of the layer (25) to be photographed.

9. Dental X-ray diagnostic device according to claim 7 or 8, characterized in that the computing device (26), proceeding from a form (25) of a layer to be photographed, processes it in the sense of a compression and/or extension and the means (20, 30) of the input device (19) for the graphic representation of forms (25, 25a, 25b, 25c, 25d) represent the form (25a, 25b, 25c, 25d) obtained in this way.

## Revendications

1. Appareil de radiodiagnostic dentaire pour la réalisation de radiographies d'ensemble de couches s'étendant à l'intérieur du crâne (1) d'un patient, notamment de couches s'étendant dans la zone de la mâchoire, et comportant
a) une unité d'enregistrement (3), qui est montée de manière à pouvoir tourner autour d'un axe vertical (2 ou 15) et sur laquelle sont fixés une source de rayonnement X (9) et, en position diamétralement opposée à cette source, un support de film (10) mobile par rapport à la source de rayonnement (9),
b) un dispositif (12,13) de maintien de la tête, qui est disposé entre la source de rayonnement (9) et le support de film (10) et au moyen duquel la tête (1) du patient peut être maintenue fixe pendant un enregistrement,
c) un premier moyen de réglage (M_{α}) destiné à déplacer l'unité d'enregistrement (3) autour de l'axe vertical (2 ou 15),
d) un second moyen de réglage (M_{β}) destiné à régler le support de film (10) par rapport à la source de rayonnement (9),
e) au moins un autre moyen de réglage (Mₓ, M_{y}) destiné à déplacer l'unité d'enregistrement (3) dans un plan perpendiculaire à l'axe vertical (2 ou 15),
f) un dispositif de commande (16) destiné à commander la source de rayonnement (9) et les moyens de réglage (Mₓ, M_{y} ainsi que M_{α}) indiqués précédemment, et
g) un dispositif d'entrée (19,23), qui fournit au dispositif de commande (16) des signaux électriques, qui correspondent à la courbe d'une couche devant être enregistrée,
caractérisé par le fait que
h) le dispositif d'entrée (19,23) comporte des moyens (20, 21; 28, 31, 32; 29,33; 30) destinés à l'entrée graphique d'au moins un tracé de courbe continu, pouvant être choisi à volonté, d'une couche devant être enregistrée ainsi qu'à la conversion du tracé de courbe choisi en des signaux électriques, et
i) il est prévu une mémoire (27) qui est reliée au dispositif d'entrée (19,23) et au dispositif de commande (16) et dans laquelle les signaux électriques, fournis à partir de l'entrée graphique, sont mémorisés en tant que données, et
j) il est prévu une unité de commande centrale (22), qui est reliée au dispositif de commande (16) et à la mémoire (27) qui est connectée à un clavier (18) et qui, lors de l'activation par appel des données à partir de la mémoire (27), commande tout d'abord le dispositif de commande (16) pour qu'il règle les moyens de réglage de telle sorte que l'unité d'enregistrement (3) et le support de film (10) prennent une position initiale, qui correspond à la couche devant être enregistrée et qui, ensuite, met en service la source de rayonnement (9) et, en fonction de la forme de l'entrée graphique de la couche devant être enregistrée, règle les moyens de réglage de telle sorte que l'unité d'enregistrement (3) et le support de film (10) exécutent les déplacements nécessaires pour la réalisation de la radiographie d'ensemble.

2. Appareil de radiodiagnostic dentaire suivant la revendication 1, caractérisé par le fait que le dispositif d'entrée (19) comporte une "Computer-Graphics-Tablet" (28), (tablette graphique à ordinateur).

3. Appareil de radiodiagnostic dentaire suivant la revendication 1, caractérisé par le fait que le dispositif d'entrée (19) comporte un moniteur (20) équipé d'un photostyle (21).

4. Appareil de radiodiagnostic dentaire suivant l'une des revendications 1 à 3, caractérisé par le fait que le dispositif d'entrée (19) comporte des moyens (20,30) pour la représentation graphique de courbes (25,25a,25b,25c,25d) de couches devant être enregistrées.

5. Appareil de radiodiagnostic dentaire suivant la revendication 4, caractérisé par le fait que la mémoire (27) est agencée de telle sorte que des données de plusieurs courbes (25) de couches devant être enregistrées peuvent être mémorisées dans la mémoire, et qu'une partie de la mémoire (27) est sous la forme d'une mémoire morte (ROM), dans laquelle sont mémorisées les données d'un nombre de courbes standards de couches devant être enregistrées.

6. Appareil de radiodiagnostic dentaire suivant la revendication 4 ou 5, caractérisé par le fait que les moyens (20) du dispositif d'entrée (19) sont agencés pour la représentation graphique de courbes (25,25a,25b,25c,25d) de couches devant être enregistrées, de telle sorte que plusieurs courbes (25,25a,25b,25c,25d) peuvent être simultanément représentées graphiquement et que le dispositif d'entrée (19) lui-même est agencé de telle sorte que l'une des courbes représentées (25,25a,25b,25c,25d) de couches devant être enregistrées peut être directement choisie pour la réalisation d'une prise de vue correspondante.

7. Appareil de radiodiagnostic dentaire suivant l'une des revendications 1 à 6, caractérisé par le fait qu'il est prévu un dispositif de calcul (26), qui convertit une courbe (25), introduite dans le dispositif d'entrée (19), d'une couche devant être enregistrée, en des données de préférence numériques pour le dispositif de commande (16).

8. Appareil de radiodiagnostic dentaire suivant l'une des revendications 4 et 7, caractérisé par le fait que le dispositif de calcul (26) détermine, à partir d'une courbe (25) d'une couche devant être enregistrée, la courbe de l'épaisseur de couche (34) associée, dont l'image peut être formée de façon nette, et les moyens (20,30) du dispositif d'entrée (19) servant à donner la représentation graphique de courbes (25,25a,25b,25c,25d) de couches devant être enregistrées, représentent ces courbes, en plus de la courbe proprement dite de la couche (25) devant être enregistrée.

9. Appareil de radiodiagnostic dentaire suivant la revendication 7 ou 8, caractérisé par le fait qu'à partir d'une courbe (25) d'une couche devant être enregistrée, le dispositif de calcul (26) traite cette courbe dans le sens d'une compression et/ou d'une dilatation, et les moyens (20,30) du dispositif d'entrée (19) utilisés pour la représentation graphique de courbes (25,25a,25b,25c,25d) représentent la courbe (25a,25b,25c,25d) ainsi obtenue.
